# EUROPEAN PATENT SPECIFICATION

(11) **EP 4 753 635 B1**
(45) Date of publication and mention of the grant of the patent: **15.07.2026**
(21) Application number: 24751810.3
(22) Date of filing: 26.07.2024
(51) Int. Cl.: A61F 5/445, A61F 5/451

(54) **A URINE COLLECTION BAG, A CATHETER SLEEVE AND A METHOD OF MANUFACTURING A URINE COLLECTION BAG - ALL WITH A TRILAYER LAMINATE FILM**
EIN URINSAMMELBEUTEL, EINE KATHETERHÜLLE UND EIN VERFAHREN ZUR HERSTELLUNG EINES URINSAMMELBEUTELS - JEWEILS MIT EINER DREISCHICHTIGEN LAMINATFOLIE
UN SAC DE COLLECTE D'URINE, UNE GAINE DE CATHÉTER ET UN PROCÉDÉ DE FABRICATION D'UN SAC DE COLLECTE D'URINE - TOUS AVEC UN FILM STRATIFIÉ TRICOUCHE

(30) Priority: 31.07.2023 US 202363516577 P; 08.09.2023 GB 202313733
(43) Date of publication of application: 10.06.2026
(73) Proprietor: ConvaTec Limited, Deeside, Flintshire CH5 2NU (GB)
(72) Inventor: YOUNG, Alice, Deeside Flintshire CH5 2NU (GB); ROBERTS, Gavin, Deeside Flintshire CH5 2NU (GB)
(74) Representative: Wilson Gunn
(86) International application number: PCT/GB2024/051962
(87) International publication number: WO 2025/027291

(56) References cited:
- AU-A1- 2010 322 259
- US-A1- 2021 100 680
- US-A1- 2021 369 486

## Description

### Technical Field of the Invention

The present invention relates to urine collection bags and catheter sleeves comprising a laminate film material.

### Background to the Invention

Urinary catheterisation is a common medical procedure used for draining urine from patients who are unable to do so naturally. Typically, a catheter is inserted into the patient's urethra and connected to a urine collection bag, allowing for the collection and monitoring of urine output. In order to ensure patient comfort and hygiene, it is crucial to develop catheter kits with components, such as urine collection bags and catheter sleeves, that offer a range of beneficial features.

One important consideration in designing catheter kit components is the choice of materials. Conventional urine collection bags and catheter sleeves are often made of thick plastic materials, which can result in bulkiness, increased weight, and discomfort for the patient. Additionally, the use of large amounts of plastic contributes to environmental concerns. Conventional materials are often known to suffer from further disadvantages including that they may experience discolouration or degradation when exposed to sterilisation methods such as steam or chemical agents. Further, conventional materials are known to produce disruptive sounds during movement, such as rustling sounds, reducing patient comfort and privacy during the catheterisation process.

AU 2010 322 259 A1 discloses a film having at least one quiet layer that comprises a polymer resin and from about 5 to 50 weight percent of a styrene-vinyl polyisoprene-styrene block-co-polyisoprene block triblock polymer.

There exists a need for new and improved materials for catheterisation applications, which overcome or mitigate one or more problems of the prior art mentioned above.

It is an aim of embodiments of the present invention to overcome or mitigate at least one problem of the prior art, whether expressly described herein or not.

### Summary of the Invention

According to a first aspect of the invention, there is provided a urine collection bag comprising a cavity for containing urine, the bag comprising a trilayer laminate film, the film comprising in order: a first ethylene-vinyl acetate layer, a polyethylene layer, and a second ethylene-vinyl acetate layer.

Such a trilayer laminate film provides several advantages over traditional materials. The layer combination employed in the trilayer film confers exceptional strength to the material, even in the case of thin films. As such, even a thin layer of the trilayer film may be successfully used in construction of catheter kit components, thus significantly reducing the amount of plastic required in manufacture. This reduction in plastic usage helps minimise waste and contributes to environmental sustainability. The film is also lightweight without any compromise to strength, and so allows for development of lightweight urine collection bags that can still hold large amounts of urine effectively. The film is also relatively quiet when handled or in transport and does not produce significant rustling or other disruptive sounds during movement, which enhances patient comfort and privacy. Additionally, the trilayer laminate film is also believed to provide excellent resistance to discolouration when subjected to sterilisation processes. Sterilisation is a critical step to maintain the hygiene and safety of catheter kit components and the film is believed to preserve its appearance and integrity even after repeated sterilisation cycles.

In some embodiments, at least 50 wt.% of the bag may comprise the trilayer film, or at least 60, 70, 80, 90, or at least 96 wt.% of the bag may comprise the trilayer film. Preferably, the bag may be made entirely of the trilayer film.

In some embodiments, the cavity of the bag is defined by the trilayer film.

In some embodiments, the bag comprises a front wall and a rear wall, wherein the front and rear walls are sealed together to form the cavity of the bag therebetween, and wherein the front and rear wall comprise the trilayer film.

In some embodiments, the front and rear walls are sealed together at their peripheries to form the cavity. The front and rear walls may be welded together at their peripheries to form the cavity. The weld may pass through all the layers of the films of the front and rear walls.

In some embodiments, the front or rear wall comprises an inlet for receiving urine.

In some embodiments, the bag comprises an attachment element for attaching the bag to a urinary catheter. In some embodiments, the attachment element comprises a mechanical fastening means. The attachment element may comprise a non-return valve to prevent flow of urine from the bag back into the catheter, in use.

The bag may be configured to hold at least 400 mL of urine, or at least 450, 500, 550, 600, 650, 700, or at least 750 mL of urine. The bag may be configured to hold between 500-2000 mL of urine, or between 750-1500 mL of urine.

In some embodiments, at least one layer of the film has a thickness of at least 2 microns, or at least 3, 4, 5, 6, 7, 8, 9 or at least 10 microns. In some embodiments, at least one layer of the film has a thickness of no greater than 80 microns, 75, 70, 65, 60, 55, 50, 45, or no greater than 40 microns. At least one layer of the film may have a thickness of between 10-40 microns, or between 15-35, or between 20-30 microns.

In some embodiments, at least one ethylene-vinyl acetate layer of the film has a thickness of at least 2 microns, or at least 3, 4, 5, 6, 7, 8, 9 or at least 10 microns. In some embodiments, at least one ethylene-vinyl acetate layer of the film has a thickness of no greater than 80 microns, 75, 70, 65, 60, 55, 50, 45, or no greater than 40 microns. At least one ethylene-vinyl acetate layer of the film may have a thickness of between 10-40 microns, or between 15-35, or between 20-30 microns. The first and/or second ethylene-vinyl acetate layer may have a thickness as described above.

In some embodiments, the polyethylene layer of the film has a thickness of at least 2 microns, or at least 3, 4, 5, 6, 7, 8, 9 or at least 10 microns. In some embodiments, the polyethylene layer of the film has a thickness of no greater than 80 microns, 75, 70, 65, 60, 55, 50, 45, or no greater than 40 microns. The polyethylene layer of the film may have a thickness of between 10-40 microns, or between 15-35, or between 20-35 microns, or between 25-35 microns.

In some embodiments, the first and second ethylene-vinyl acetate layers have a thickness of between 10-40 microns each; and the polyethylene layer has a thickness of between 10-40 microns, or between 15-35, or between 20-35 microns, or between 25-35 microns.

In some embodiments, the first and second ethylene-vinyl acetate layers have a thickness of between 15-35 microns each; and the polyethylene layer has a thickness of between 10-40 microns, or between 15-35, or between 20-35 microns, or between 25-35 microns.

In some embodiments, the first and second ethylene-vinyl acetate layers have a thickness of between 20-30 microns each; and the polyethylene layer has a thickness of between 10-40 microns, or between 15-35, or between 20-35 microns, or between 25-35 microns.

In some embodiments, the film has a total thickness of at least 40 microns, or at least 45, or at least 50 microns. The film may have a total thickness of no greater than 150 microns, or no greater than 145, 140, 135, 130, 125, 120, 115, 110, 105, or no greater than 100 microns. The film may have a total thickness of between 40-100 microns, or between 45-100, or between 50-100 microns, or between 55-95, or preferably between 60-90, 65-85, or preferably between 70-80 microns.

In some embodiments, the film may have a total thickness of between 40-95 microns, or between 45-90 microns.

In some embodiments, both the first and second ethylene-vinyl acetate layers of the film have the same thickness. The first and/or second ethylene-vinyl acetate layer may have the same thickness as the polyethylene layer. In some embodiments, all three layers of the trilayer film have the same thickness.

In other embodiments, the ethylene-vinyl acetate layers of the film have a different thickness to the polyethylene layer. The polyethylene layer may be thicker than the ethylene-vinyl acetate layers. In some embodiments, all three layers of the trilayer film have a different thickness.

The polyethylene layer may comprise polyethylene in a total amount of at least 80 wt.% of the polyethylene layer, or at least 85, 90, or at least 95 wt.% of the polyethylene layer. In some embodiments, the polyethylene layer is entirely made of polyethylene.

In some preferred embodiments, there is provided a urine collection bag comprising a cavity for containing urine, the bag comprising a trilayer laminate film, the film comprising in order: a first ethylene-vinyl acetate layer, a polyethylene layer, and a second ethylene-vinyl acetate layer, wherein the polyethylene layer of the film comprises polyethylene in a total amount of at least 80 wt.% of the polyethylene layer, and preferably the polyethylene layer is entirely made of polyethylene.

In some preferred embodiments, the polyethylene layer of the film comprises ethylene-vinyl acetate in a total amount of less than 10 wt.% of the polyethylene layer, or less than 5 wt.% of the polyethylene layer. In preferred embodiments, the polyethylene layer does not comprise ethylene-vinyl acetate.

In some preferred embodiments, there is provided a urine collection bag comprising a cavity for containing urine, the bag comprising a trilayer laminate film, the film comprising in order: a first ethylene-vinyl acetate layer, a polyethylene layer, and a second ethylene-vinyl acetate layer, wherein the polyethylene layer of the film comprises ethylene-vinyl acetate in a total amount of less than 10 wt.% of the polyethylene layer, and preferably the polyethylene layer does not comprise ethylene-vinyl acetate

At least one ethylene-vinyl acetate layer may comprise ethylene-vinyl acetate in a total amount of at least 80 wt.% of the ethylene-vinyl acetate layer, or at least 85, 90, or at least 95 wt.% of the ethylene-vinyl acetate layer. In some embodiments, the ethylene-vinyl acetate layer is entirely made of ethylene-vinyl acetate.

In some preferred embodiments, there is provided a urine collection bag comprising a cavity for containing urine, the bag comprising a trilayer laminate film, the film comprising in order: a first ethylene-vinyl acetate layer, a polyethylene layer, and a second ethylene-vinyl acetate layer, wherein at least one ethylene-vinyl acetate layer of the film comprises ethylene-vinyl acetate in a total amount of at least 95 wt.% of the ethylene-vinyl acetate layer, and preferably the at least one ethylene-vinyl acetate layer is entirely made of ethylene-vinyl acetate.

In some preferred embodiments, at least one ethylene-vinyl acetate layer of the film comprises polyethylene in a total amount of less than 10 wt.% of the ethylene-vinyl acetate layer, or less than 5 wt.% of the ethylene-vinyl acetate layer. In preferred embodiments, at least one ethylene-vinyl acetate layer and preferably both the first and second ethylene-vinyl acetate layers does not comprise polyethylene.

In some preferred embodiments, there is provided a urine collection bag comprising a cavity for containing urine, the bag comprising a trilayer laminate film, the film comprising in order: a first ethylene-vinyl acetate layer, a polyethylene layer, and a second ethylene-vinyl acetate layer, wherein at least one ethylene-vinyl acetate layer of the film comprises polyethylene in a total amount of less than 10 wt.% of the ethylene-vinyl acetate layer, and preferably the at least one ethylene-vinyl acetate layer does not comprise polyethylene.

In some preferred embodiments, the first and second ethylene-vinyl acetate layers of the film are each entirely made of ethylene-vinyl acetate, and the polyethylene layer of the film is entirely made of polyethylene.

In some preferred embodiments, there is provided a urine collection bag comprising a cavity for containing urine, the bag comprising a trilayer laminate film, the film comprising in order: a first ethylene-vinyl acetate layer, a polyethylene layer, and a second ethylene-vinyl acetate layer, wherein the first and second ethylene-vinyl acetate layers of the film are each entirely made of ethylene-vinyl acetate, and the polyethylene layer of the film is entirely made of polyethylene.

It has been found that providing a layered film structure in which ethylene-vinyl acetate is largely separated from polyethylene, and in which both materials are present in discrete layers with the polyethylene layer sandwiched between ethylene-vinyl acetate, allows for a film that not only has excellent strength, but which is also quiet when handled and produces minimal rustling or other disruptive sounds. In addition, such a film structure provides excellent resistance to discolouration even when the film is subjected to multiple sterilisation processes.

In some embodiments, the film comprises polyethylene in a total amount of at least 2 wt.%, or at least 3, 4, or at least 5 wt.%. In some embodiments, the film comprises polyethylene in a total amount of no greater than 80 wt.%, 75, or no greater 70 wt.%, or no greater than 60, 50, 40, 30, 20, or no greater than 15 wt. %.

In some embodiments, the film comprises polyethylene in a total amount of between 5-70 wt.%, or between 10-70, or between 15-70, or between 20-70, or between 30-60, or between 30-50 wt.%. In other embodiments, the film comprises polyethylene in a total amount of between 2-20 wt.%, or between 5-15, or between 7-13 wt.%.

In some preferred embodiments, the film comprises polyethylene in a total amount of between 5-50 wt.%, or preferably between 10-45 wt.%.

In some preferred embodiments, there is provided a urine collection bag comprising a cavity for containing urine, the bag comprising a trilayer laminate film, the film comprising in order: a first ethylene-vinyl acetate layer, a polyethylene layer, and a second ethylene-vinyl acetate layer, wherein the film comprises polyethylene in a total amount of between 5-50 wt.%, or preferably between 10-45 wt.%.

In some embodiments, the film comprises ethylene-vinyl acetate in a total amount of at least 2 wt.%, or at least 3, 4, 5, 10, 15, 20, or at least 25 wt.%. In some embodiments, the film comprises ethylene-vinyl acetate in a total amount of no greater than 95 wt.%, or no greater than 90, 85, 80, 75, or no greater 70 wt.%, or no greater than 60, 50, 40, 30, 20, or no greater than 15 wt.%.

In some embodiments, the film comprises ethylene-vinyl acetate in a total amount of between 5-70 wt.%, or between 10-70, or between 15-70, or between 20-70, or between 30-60, or between 30-50 wt.%. In other embodiments, the film comprises ethylene-vinyl acetate in a total amount of between 80-98 wt.%, or between 75-95, or between 87-93 wt.%.

In some preferred embodiments, the film comprises ethylene-vinyl acetate in a total amount of between 50-95 wt.%, or preferably between 55-90 wt.%.

In some preferred embodiments, there is provided a urine collection bag comprising a cavity for containing urine, the bag comprising a trilayer laminate film, the film comprising in order: a first ethylene-vinyl acetate layer, a polyethylene layer, and a second ethylene-vinyl acetate layer, wherein the film comprises ethylene-vinyl acetate in a total amount of between 50-95 wt.%, or preferably between 55-90 wt.%.

In some embodiments, the film comprises polyethylene in a total amount of between 10-70 wt.% and ethylene-vinyl acetate in a total amount of between 30-90 wt.%. The film may comprise polyethylene in a total amount of between 20-60 wt.% and ethylene-vinyl acetate in a total amount of between 40-80 wt.%. The film may comprise polyethylene in a total amount of between 30-50 wt.% and ethylene-vinyl acetate in a total amount of between 50-70 wt.%.

In other embodiments, the film comprises polyethylene in a total amount of between 2-20 wt.% and the film comprises ethylene-vinyl acetate in a total amount of between 80-98 wt.%. The film may comprise polyethylene in a total amount of between 5-15 wt.% and the film may comprise ethylene-vinyl acetate in a total amount of between 85-95 wt.%. The film may comprise polyethylene in a total amount of between 7-13 wt.% and the film may comprise ethylene-vinyl acetate in a total amount of between 87-93 wt.%.

In some embodiments, the film has a total thickness of between 50-100 microns; and the film comprises polyethylene in a total amount of between 5-70 wt.%, or between 10-70, or between 15-70, or between 20-70, or between 30-60, or between 30-50 wt.%. In other embodiments, the film may have a total thickness of between 50-100 microns; and the film may comprise polyethylene in a total amount of between 2-20 wt.%, or between 5-15, or between 7-13 wt.%.

In some embodiments, the film has a total thickness of between 60-90 microns; and the film comprises polyethylene in a total amount of between 5-70 wt.%, or between 10-70, or between 15-70, or between 20-70, or between 30-60, or between 30-50 wt.%. In other embodiments, the film may have a total thickness of between 60-90 microns; and the film may comprise polyethylene in a total amount of between 2-20 wt.%, or between 5-15, or between 7-13 wt.%.

In some embodiments, the film has a total thickness of between 70-80 microns; and the film comprises polyethylene in a total amount of between 5-70 wt.%, or between 10-70, or between 15-70, or between 20-70, or between 30-60, or between 30-50 wt.%. In other embodiments, the film may have a total thickness of between 70-80 microns; and the film may comprise polyethylene in a total amount of between 2-20 wt.%, or between 5-15, or between 7-13 wt.%.

In some embodiments, the polyethylene layer comprises low-density polyethylene. The polyethylene layer may comprise linear low-density polyethylene.

In some embodiments, the ethylene-vinyl acetate of the first and/or second ethylene-vinyl acetate layer may have a vinyl acetate content of between 10-20% of the copolymer, or between 15-20% of the copolymer.

In some preferred embodiments, the trilayer film is symmetrical. In other embodiments, the first ethylene-vinyl acetate layer may be thicker or thinner than the second ethylene-vinyl acetate layer.

In some embodiments, the trilayer film is a cast film. Alternatively, the trilayer film may be a blown film.

According to a second aspect of the invention, there is provided a catheter sleeve comprising a trilayer laminate film comprising in order: a first ethylene-vinyl acetate layer, a polyethylene layer, and a second ethylene-vinyl acetate layer.

The trilayer laminate film of the second aspect of the invention may be the trilayer film of the first aspect of the invention above. Statements of invention above relating to the trilayer film of the first aspect of the invention may be applied *mutatis mutandis* to the second aspect of the invention. Statements of invention below for the second aspect of the invention may also be applied *mutatis mutandis* to the other aspects of the invention.

In some embodiments, the sleeve may comprise a tubular body comprising the trilayer film and which tubular body comprises a hollow lumen extending longitudinally therethrough. The hollow lumen may preferably be adapted to receive a catheter, in use.

In some embodiments, at least 50 wt.% of the sleeve may comprise the trilayer film, or at least 60, 70, 80, 90, or at least 96 wt.% of the sleeve may comprise the trilayer film. Preferably, the sleeve may be made entirely of the trilayer film.

Film thicknesses for the sleeve of the second aspect of the invention may be as described for the bag of the first aspect of the invention. Alternatively, the film of the sleeve of the second aspect of the invention may have a different thickness to the film of the bag of the first aspect of the invention. The film of the sleeve of the second aspect of the invention may be thinner than the film of the bag of the first aspect of the invention.

In some embodiments, at least one layer of the film has a thickness of at least 1 micron, or at least 2, 3, 4, or at least 5 microns. In some embodiments, at least one layer of the film has a thickness of no greater than 50 microns, or no greater than 45, 40, 35, or no greater than 30 microns. At least one layer of the film may have a thickness of between 5-30 microns, or between 10-25, or between 15-20 microns.

In some embodiments, at least one ethylene-vinyl acetate layer of the film has a thickness of at least 1 micron, or at least 2, 3, 4, or at least 5 microns. In some embodiments, at least one ethylene-vinyl acetate layer of the film has a thickness of no greater than 50 microns, or no greater than 45, 40, 35, or no greater than 30 microns. At least one ethylene-vinyl acetate layer of the film may have a thickness of between 5-30 microns, or between 10-25, or between 10-20 microns. The first and/or second ethylene-vinyl acetate layer may have a thickness as described above.

In some embodiments, the polyethylene layer of the film has a thickness of at least 1 micron, or at least 2, 3, 4, or at least 5 microns. In some embodiments, the polyethylene layer of the film has a thickness of no greater than 60 microns, or no greater than 55, 50, 45, 40, 35, or no greater than 30 microns. The polyethylene layer of the film may have a thickness of between 5-30 microns, or between 10-25, or between 15-25 microns.

In some embodiments, the film has a total thickness of at least 20 microns, or at least 25, 30, 35, or at least 40 microns. The film may have a total thickness of no greater than 100 microns, or no greater than 90, 80, or no greater than 70 microns. The film may have a total thickness of between 40-95 microns, or between 40-90 microns, or between 40-70 microns, or between 45-65, 45-60, or preferably between 45-55 microns.

In some embodiments, the film has a total thickness of between 40-95 microns, or between 45-90 microns.

In some embodiments, both the first and second ethylene-vinyl acetate layers of the film have the same thickness. The first and/or second ethylene-vinyl acetate layer may have the same thickness as the polyethylene layer. In some embodiments, all three layers of the trilayer film have the same thickness.

In other embodiments, the ethylene-vinyl acetate layers of the film have a different thickness to the polyethylene layer. The polyethylene layer may be thicker than the ethylene-vinyl acetate layers. In some embodiments, all three layers of the trilayer film have a different thickness.

In some embodiments, the film has a total thickness of between 40-70 microns; and the film comprises polyethylene in a total amount of between 5-70 wt.%, or between 10-70, or between 15-70, or between 20-70, or between 30-60, or between 30-50 wt.%. In other embodiments, the film may have a total thickness of between 40-70 microns; and the film may comprise polyethylene in a total amount of between 2-20 wt.%, or between 5-15, or between 7-13 wt.%.

In some embodiments, the film has a total thickness of between 45-65 microns; and the film comprises polyethylene in a total amount of between 5-70 wt.%, or between 10-70, or between 15-70, or between 20-70, or between 30-60, or between 30-50 wt.%. In other embodiments, the film may have a total thickness of between 45-65 microns; and the film may comprise polyethylene in a total amount of between 2-20 wt.%, or between 5-15, or between 7-13 wt.%.

In some embodiments, the film has a total thickness of between 45-60 microns; and the film comprises polyethylene in a total amount of between 5-70 wt.%, or between 10-70, or between 15-70, or between 20-70, or between 30-60, or between 30-50 wt.%. In other embodiments, the film may have a total thickness of between 45-60 microns; and the film may comprise polyethylene in a total amount of between 2-20 wt.%, or between 5-15, or between 7-13 wt.%.

In some embodiments, the film has a total thickness of between 45-55 microns; and the film comprises polyethylene in a total amount of between 5-70 wt.%, or between 10-70, or between 15-70, or between 20-70, or between 30-60, or between 30-50 wt.%. In other embodiments, the film may have a total thickness of between 45-55 microns; and the film may comprise polyethylene in a total amount of between 2-20 wt.%, or between 5-15, or between 7-13 wt.%.

In some preferred embodiments, there is provided a catheter sleeve comprising a trilayer laminate film comprising in order: a first ethylene-vinyl acetate layer, a polyethylene layer, and a second ethylene-vinyl acetate layer, wherein the polyethylene layer of the film comprises polyethylene in a total amount of at least 80 wt.% of the polyethylene layer, and preferably the polyethylene layer is entirely made of polyethylene.

In some preferred embodiments, there is provided a catheter sleeve comprising a trilayer laminate film comprising in order: a first ethylene-vinyl acetate layer, a polyethylene layer, and a second ethylene-vinyl acetate layer, wherein the polyethylene layer of the film comprises ethylene-vinyl acetate in a total amount of less than 10 wt.% of the polyethylene layer, and preferably the polyethylene layer does not comprise ethylene-vinyl acetate.

In some preferred embodiments, there is provided a catheter sleeve comprising a trilayer laminate film comprising in order: a first ethylene-vinyl acetate layer, a polyethylene layer, and a second ethylene-vinyl acetate layer, wherein at least one ethylene-vinyl acetate layer of the film comprises ethylene-vinyl acetate in a total amount of at least 95 wt.% of the ethylene-vinyl acetate layer, and preferably the at least one ethylene-vinyl acetate layer is entirely made of ethylene-vinyl acetate.

In some preferred embodiments, there is provided a catheter sleeve comprising a trilayer laminate film comprising in order: a first ethylene-vinyl acetate layer, a polyethylene layer, and a second ethylene-vinyl acetate layer, wherein at least one ethylene-vinyl acetate layer of the film comprises polyethylene in a total amount of less than 10 wt.% of the ethylene-vinyl acetate layer, and preferably the at least one ethylene-vinyl acetate layer does not comprise polyethylene.

In some preferred embodiments, there is provided a catheter sleeve comprising a trilayer laminate film comprising in order: a first ethylene-vinyl acetate layer, a polyethylene layer, and a second ethylene-vinyl acetate layer, wherein the first and second ethylene-vinyl acetate layers of the film are each entirely made of ethylene-vinyl acetate, and the polyethylene layer of the film is entirely made of polyethylene.

In some preferred embodiments, there is provided a catheter sleeve comprising a trilayer laminate film comprising in order: a first ethylene-vinyl acetate layer, a polyethylene layer, and a second ethylene-vinyl acetate layer, wherein the film comprises polyethylene in a total amount of between 5-50 wt.%, or preferably between 10-45 wt.%.

In some preferred embodiments, there is provided a catheter sleeve comprising a trilayer laminate film comprising in order: a first ethylene-vinyl acetate layer, a polyethylene layer, and a second ethylene-vinyl acetate layer, wherein the film comprises ethylene-vinyl acetate in a total amount of between 50-95 wt.%, or preferably between 55-90 wt.%.

According to third aspect of the invention, there is provided a urinary catheter kit comprising: a catheter comprising a proximal end for insertion into the body and a distal end; a urine collection bag comprising an inlet adapted for fluid communication with the catheter distal end; and a catheter sleeve configured to enclose at least part of the catheter length from the proximal end to the distal end, wherein the urine collection bag and catheter sleeve comprise a trilayer laminate film, the film comprising in order: a first ethylene-vinyl acetate layer, a polyethylene layer, and a second ethylene-vinyl acetate layer.

The urine collection bag of the third aspect of the invention may be the urine collection bag of the first aspect of the invention.

The sleeve of the third aspect of the invention may be the sleeve of the second aspect of the invention.

The trilayer film of the third aspect of the invention may preferably be the trilayer film of the first and/or second aspect of the invention.

Statements of invention above for the first and second aspects of the invention may also be applied *mutatis mutandis* to the third aspect of the invention.

The trilayer film of the sleeve may be identical to the trilayer film of the bag. Alternatively, the trilayer film of the sleeve may have a different film thickness to the trilayer film of the bag. The trilayer film of the sleeve may be thinner than the trilayer film of the bag. The trilayer film of the bag may have a film thickness as described in statements of invention for the first aspect of the invention above. The trilayer film of the sleeve may have a film thickness as described in statements of invention for the second aspect of the invention above.

In some embodiments, the sleeve may be configured to enclose at least 5% of the length of the catheter from the proximal end of the catheter to the distal end, or at least 10, 20, 30, 40 or at least 50% of the length of the catheter from the proximal end of the catheter to the distal end.

In some embodiments, the sleeve may be configured to enclose the entire length of the catheter from the proximal end to the distal end. In some embodiments, the sleeve may not enclose the entire length of the catheter from the proximal end to the distal end. In such embodiments, the sleeve may be slidable along the entire length of the catheter, in use.

The catheter may be an intermittent catheter. Such a catheter is typically inserted into a body for short time periods, such as less than a day. Alternatively, the catheter may be an indwelling (Foley) catheter. Such a catheter is typically inserted and kept in a body for long periods of time, such as several days to months.

The catheter may be a single use catheter or a multiple use catheter.

In some embodiments, the catheter comprises a hollow tubular body, preferably a hollow polymeric tubular body. The hollow polymeric tubular body may comprise a base polymer.

In some embodiments, the catheter further comprises at least one additive, preferably at least one lubricious additive. Preferably, the hollow tubular body comprises at least one additive.

In some embodiments, at least one additive is a hydrophilic or amphiphilic additive.

In some preferred embodiments, there is provided a urinary catheter kit comprising: a catheter comprising a proximal end for insertion into the body and a distal end; a urine collection bag comprising an inlet adapted for fluid communication with the catheter distal end; and a catheter sleeve configured to enclose at least part of the catheter length from the proximal end to the distal end, wherein the urine collection bag and catheter sleeve comprise a trilayer laminate film, the film comprising in order: a first ethylene-vinyl acetate layer, a polyethylene layer, and a second ethylene-vinyl acetate layer, wherein the polyethylene layer of the film comprises polyethylene in a total amount of at least 80 wt.% of the polyethylene layer, and preferably the polyethylene layer is entirely made of polyethylene.

In some preferred embodiments, there is provided a urinary catheter kit comprising: a catheter comprising a proximal end for insertion into the body and a distal end; a urine collection bag comprising an inlet adapted for fluid communication with the catheter distal end; and a catheter sleeve configured to enclose at least part of the catheter length from the proximal end to the distal end, wherein the urine collection bag and catheter sleeve comprise a trilayer laminate film, the film comprising in order: a first ethylene-vinyl acetate layer, a polyethylene layer, and a second ethylene-vinyl acetate layer, wherein the polyethylene layer of the film comprises ethylene-vinyl acetate in a total amount of less than 10 wt.% of the polyethylene layer, and preferably the polyethylene layer does not comprise ethylene-vinyl acetate.

In some preferred embodiments, there is provided a urinary catheter kit comprising: a catheter comprising a proximal end for insertion into the body and a distal end; a urine collection bag comprising an inlet adapted for fluid communication with the catheter distal end; and a catheter sleeve configured to enclose at least part of the catheter length from the proximal end to the distal end, wherein the urine collection bag and catheter sleeve comprise a trilayer laminate film, the film comprising in order: a first ethylene-vinyl acetate layer, a polyethylene layer, and a second ethylene-vinyl acetate layer, wherein at least one ethylene-vinyl acetate layer of the film comprises ethylene-vinyl acetate in a total amount of at least 95 wt.% of the ethylene-vinyl acetate layer, and preferably the at least one ethylene-vinyl acetate layer is entirely made of ethylene-vinyl acetate.

In some preferred embodiments, there is provided a urinary catheter kit comprising: a catheter comprising a proximal end for insertion into the body and a distal end; a urine collection bag comprising an inlet adapted for fluid communication with the catheter distal end; and a catheter sleeve configured to enclose at least part of the catheter length from the proximal end to the distal end, wherein the urine collection bag and catheter sleeve comprise a trilayer laminate film, the film comprising in order: a first ethylene-vinyl acetate layer, a polyethylene layer, and a second ethylene-vinyl acetate layer, wherein at least one ethylene-vinyl acetate layer of the film comprises polyethylene in a total amount of less than 10 wt.% of the ethylene-vinyl acetate layer, and preferably the at least one ethylene-vinyl acetate layer does not comprise polyethylene.

In some preferred embodiments, there is provided a urinary catheter kit comprising: a catheter comprising a proximal end for insertion into the body and a distal end; a urine collection bag comprising an inlet adapted for fluid communication with the catheter distal end; and a catheter sleeve configured to enclose at least part of the catheter length from the proximal end to the distal end, wherein the urine collection bag and catheter sleeve comprise a trilayer laminate film, the film comprising in order: a first ethylene-vinyl acetate layer, a polyethylene layer, and a second ethylene-vinyl acetate layer, wherein the first and second ethylene-vinyl acetate layers of the film are each entirely made of ethylene-vinyl acetate, and the polyethylene layer of the film is entirely made of polyethylene.

In some preferred embodiments, there is provided a urinary catheter kit comprising: a catheter comprising a proximal end for insertion into the body and a distal end; a urine collection bag comprising an inlet adapted for fluid communication with the catheter distal end; and a catheter sleeve configured to enclose at least part of the catheter length from the proximal end to the distal end, wherein the urine collection bag and catheter sleeve comprise a trilayer laminate film, the film comprising in order: a first ethylene-vinyl acetate layer, a polyethylene layer, and a second ethylene-vinyl acetate layer, wherein the film comprises polyethylene in a total amount of between 5-50 wt.%, or preferably between 10-45 wt.%.

In some preferred embodiments, there is provided a urinary catheter kit comprising: a catheter comprising a proximal end for insertion into the body and a distal end; a urine collection bag comprising an inlet adapted for fluid communication with the catheter distal end; and a catheter sleeve configured to enclose at least part of the catheter length from the proximal end to the distal end, wherein the urine collection bag and catheter sleeve comprise a trilayer laminate film, the film comprising in order: a first ethylene-vinyl acetate layer, a polyethylene layer, and a second ethylene-vinyl acetate layer, wherein the film comprises ethylene-vinyl acetate in a total amount of between 50-95 wt.%, or preferably between 55-90 wt. %.

According to a fourth aspect of the invention, there is provided a method of manufacturing a urine collection bag comprising the steps of:
(a) Providing a front wall and a rear wall, wherein the front and rear walls comprise a trilayer film comprising in order: a first ethylene-vinyl acetate layer, a polyethylene layer, and a second ethylene-vinyl acetate layer; and
(b) Sealing the front and rear walls together to form a cavity therebetween for containing urine;
   wherein the method further comprises providing an inlet for receiving urine in the front or rear wall before or after step (b).

The urine collection bag of the fourth aspect of the invention is preferably the urine collection bag of the first aspect of the invention. Statements of invention above for any other aspect of the invention may also be applied *mutatis mutandis* to the fourth aspect of the invention.

The method may comprise a further step prior to step (a) of forming the trilayer film of the front and/or rear wall. Forming the film of the front and/or rear wall may comprise: providing a first and second ethylene-vinyl acetate layer polymer composition; providing a polyethylene layer polymer composition; and co-extruding the first and second ethylene-vinyl acetate layer polymer compositions and the polyethylene layer polymer composition to provide the trilayer film, such that the film comprises in order: the first ethylene-vinyl acetate layer, the polyethylene layer, and the second ethylene-vinyl acetate layer. The polymer compositions may comprise polymer melts. Alternatively, the polymer compositions may comprise solid polymer sheets or pellets. The extrusion method may comprise multilayer cast extrusion.

Step (b) may comprise welding the front and rear walls together, preferably as described for the first aspect of the invention above.

The welding may employ a method that is independently selected from the group consisting of: ultrasonic welding, heat sealing, radio frequency welding, laser welding, and combinations thereof.

In some embodiments, the method comprises providing an inlet in the front or rear wall before step (b). In other embodiments, the method comprises providing an inlet in the front or rear wall after step (b). The inlet may take the form of an aperture in the front or rear wall.

The method may comprise the step of providing an attachment element in the front or rear wall for attaching the bag to a urinary catheter. The step of providing an attachment element may take place before or after step (b). In some embodiments, the attachment element comprises a mechanical fastening means. The attachment element may comprise a non-return valve to prevent flow of urine from the bag back into the catheter, in use. The non-return valve may be placed between the layers of the film. The non-return valve may be placed in an aperture comprising the inlet in the front or rear wall.

In some preferred embodiments, there is provided a method of manufacturing a urine collection bag comprising the steps of:
(a) Providing a front wall and a rear wall, wherein the front and rear walls comprise a trilayer film comprising in order: a first ethylene-vinyl acetate layer, a polyethylene layer, and a second ethylene-vinyl acetate layer, wherein the polyethylene layer of the film comprises polyethylene in a total amount of at least 80 wt.% of the polyethylene layer, and preferably the polyethylene layer is entirely made of polyethylene; and
(b) Sealing the front and rear walls together to form a cavity therebetween for containing urine;
wherein the method further comprises providing an inlet for receiving urine in the front or rear wall before or after step (b).

In some preferred embodiments, there is provided a method of manufacturing a urine collection bag comprising the steps of:
(a) Providing a front wall and a rear wall, wherein the front and rear walls comprise a trilayer film comprising in order: a first ethylene-vinyl acetate layer, a polyethylene layer, and a second ethylene-vinyl acetate layer, wherein the polyethylene layer of the film comprises ethylene-vinyl acetate in a total amount of less than 10 wt.% of the polyethylene layer, and preferably the polyethylene layer does not comprise ethylene-vinyl acetate; and
(b) Sealing the front and rear walls together to form a cavity therebetween for containing urine;
wherein the method further comprises providing an inlet for receiving urine in the front or rear wall before or after step (b).

In some preferred embodiments, there is provided a method of manufacturing a urine collection bag comprising the steps of:
(a) Providing a front wall and a rear wall, wherein the front and rear walls comprise a trilayer film comprising in order: a first ethylene-vinyl acetate layer, a polyethylene layer, and a second ethylene-vinyl acetate layer, wherein at least one ethylene-vinyl acetate layer of the film comprises ethylene-vinyl acetate in a total amount of at least 95 wt.% of the ethylene-vinyl acetate layer, and preferably the at least one ethylene-vinyl acetate layer is entirely made of ethylene-vinyl acetate; and
(b) Sealing the front and rear walls together to form a cavity therebetween for containing urine;

wherein the method further comprises providing an inlet for receiving urine in the front or rear wall before or after step (b).

In some preferred embodiments, there is provided a method of manufacturing a urine collection bag comprising the steps of:
(a) Providing a front wall and a rear wall, wherein the front and rear walls comprise a trilayer film comprising in order: a first ethylene-vinyl acetate layer, a polyethylene layer, and a second ethylene-vinyl acetate layer, wherein at least one ethylene-vinyl acetate layer of the film comprises polyethylene in a total amount of less than 10 wt.% of the ethylene-vinyl acetate layer, and preferably the at least one ethylene-vinyl acetate layer does not comprise polyethylene; and
(b) Sealing the front and rear walls together to form a cavity therebetween for containing urine;

wherein the method further comprises providing an inlet for receiving urine in the front or rear wall before or after step (b).

In some preferred embodiments, there is provided a method of manufacturing a urine collection bag comprising the steps of:
(a) Providing a front wall and a rear wall, wherein the front and rear walls comprise a trilayer film comprising in order: a first ethylene-vinyl acetate layer, a polyethylene layer, and a second ethylene-vinyl acetate layer, wherein the first and second ethylene-vinyl acetate layers of the film are each entirely made of ethylene-vinyl acetate, and the polyethylene layer of the film is entirely made of polyethylene; and
(b) Sealing the front and rear walls together to form a cavity therebetween for containing urine;
wherein the method further comprises providing an inlet for receiving urine in the front or rear wall before or after step (b).

In some preferred embodiments, there is provided a method of manufacturing a urine collection bag comprising the steps of:
(a) Providing a front wall and a rear wall, wherein the front and rear walls comprise a trilayer film comprising in order: a first ethylene-vinyl acetate layer, a polyethylene layer, and a second ethylene-vinyl acetate layer, wherein the film comprises polyethylene in a total amount of between 5-50 wt.%, or preferably between 10-45 wt.%; and
(b) Sealing the front and rear walls together to form a cavity therebetween for containing urine;
wherein the method further comprises providing an inlet for receiving urine in the front or rear wall before or after step (b).

In some preferred embodiments, there is provided a method of manufacturing a urine collection bag comprising the steps of:
(a) Providing a front wall and a rear wall, wherein the front and rear walls comprise a trilayer film comprising in order: a first ethylene-vinyl acetate layer, a polyethylene layer, and a second ethylene-vinyl acetate layer, wherein the film comprises ethylene-vinyl acetate in a total amount of between 50-95 wt.%, or preferably between 55-90 wt.%; and
(b) Sealing the front and rear walls together to form a cavity therebetween for containing urine;
wherein the method further comprises providing an inlet for receiving urine in the front or rear wall before or after step (b).

### Detailed Description of the Invention

In order that the invention may be more clearly understood embodiments thereof will now be described, by way of example only, with reference to the accompanying drawings, of which:
- Figure 1: shows a perspective view of an embodiment of a urinary catheter kit of the invention comprising an embodiment of a urine collection bag and catheter sleeve of the invention.
- Figure 2: shows a cross-sectional view through the catheter sleeve of Figure 1.
- Figure 3: shows a side cross-sectional view of the urine collection bag of Figure 1.
- Figure 4: shows a cross-sectional view of the trilayer laminate film of the catheter sleeve of Figure 1.
- Figure 5: shows a cross-sectional view of the trilayer laminate film of the urine collection bag of Figure 1.

### Urinary catheter kit of the invention

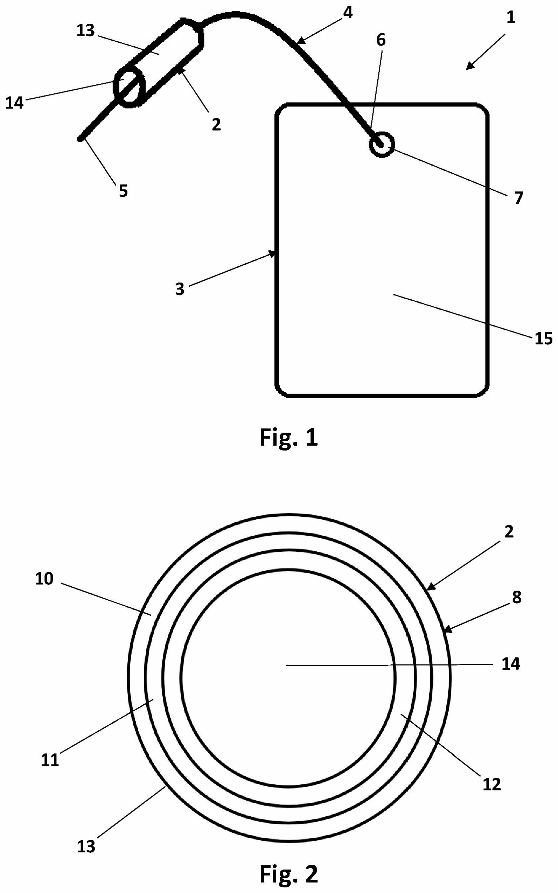

Figure 1 illustrates an embodiment of a urinary catheter kit 1 of the invention comprising an embodiment of a catheter sleeve 2 and a urine collection bag 3 of the invention.

The urinary catheter kit 1 comprises an intermittent urinary catheter 4 comprising a proximal end 5 for insertion into the body and a distal end 6. The kit 1 further comprises the urine collection bag 3 which comprises an inlet 7 adapted for fluid communication with the catheter distal end 6. The distal end 6 of the catheter 4 is attached to the bag 3 at the inlet 7. The kit 1 further comprises catheter sleeve 2 which is wrapped around and encloses approximately a third of the length of the catheter 4 from the proximal 5 to the distal end 6. The catheter sleeve 2 is slidable along the length of the catheter 4 and so can be moved from the proximal end 5 to the distal end 6 and vice versa, in use.

Both the catheter sleeve 2 and the urine collection bag 3 comprise a trilayer laminate film 8,9. The film 8 of the catheter sleeve 2 and the film 9 of the bag 3 both comprise in order: a first ethylene-vinyl acetate layer 10,100, a polyethylene layer 11,110, and a second ethylene-vinyl acetate layer 12,120.

The structure of the catheter sleeve 2 is more clearly illustrated by the cross-sectional view in Figure 2. Sleeve 2 comprises a tubular body 13 comprising the trilayer film 8. The tubular body 13 comprises a hollow lumen 14 extending longitudinally therethrough through which the catheter 4 is received, as shown in Figure 1. The sleeve 2 is formed entirely of the trilayer film 8.

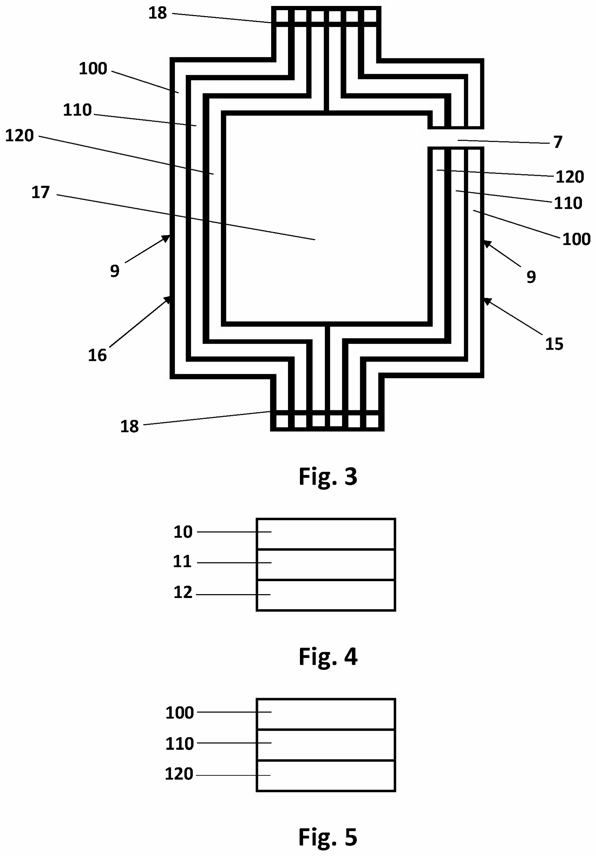

The structure of the urine collection bag 3 is more clearly illustrated by the side cross-sectional view of the bag 3 in Figure 3. Bag 3 comprises a front wall 15 and a rear wall 16. Both the front wall 15 and the rear wall 16 comprise trilayer laminate film 9. The front wall 15 and the rear wall 16 are sealed together to form a cavity 17 of the bag 3 therebetween for holding urine. The front wall 15 and the rear wall 16 are sealed together by a weld 18 formed at their peripheries to form the cavity 17 of the bag 3. The weld 18 passes through all layers of the films 9 of the front 15 and rear walls 16 in order to hold the front 15 and rear wall 16 together.

The front wall 15 of the bag 3 further comprises the inlet 7 adapted for fluid communication with the catheter distal end 6. The inlet 7 comprises an aperture formed in the front wall 15.

Urinary catheter kit 1 is used in the conventional manner.

### Structure of trilayer laminate film 8 of catheter sleeve 2

Figure 4 shows a cross-sectional view of the trilayer laminate film 8 of the catheter sleeve 2.

The trilayer film 8 has a layer structure as described in Table 1 below. The order of arrangement of the layers of the film 8 are as depicted in Table 1.

**Table 1**

| **Layer No.** | **Layer type** | **Layer materials** | **Layer thickness (µm)** |
|---|---|---|---|
| 10 | First ethylene-vinyl acetate layer | Ethylene-vinyl acetate: 100% | 20 |
| 11 | Polyethylene layer | Polyethylene: 100% | 20 |
| 12 | Second ethylene-vinyl acetate layer | Ethylene-vinyl acetate: 100% | 20 |

The percentage by weight of polyethylene in film 8 is approximately 33 wt.% and the percentage by weight of ethylene-vinyl acetate in film 8 is approximately 66 wt.%.

The trilayer film 8 is produced by multilayer cast extrusion, which involves providing in polymer melt form: first and second ethylene-vinyl acetate layer polymer compositions corresponding to layers 10 and 12, and a polyethylene layer polymer composition corresponding to layer 11. The polymer compositions for the layers of the film are then co-extruded to form the trilayer film 8.

### Structure of trilayer laminate film 9 of urine collection bag 3

Figure 5 shows a cross-sectional view of the trilayer laminate film 9 of the urine collection bag 3.

The trilayer film 9 has a layer structure as described in Table 2 below. The order of arrangement of the layers of the film 9 are as depicted in Table 2.

**Table 2**

| **Layer No.** | **Layer type** | **Layer materials** | **Layer thickness (µm)** |
|---|---|---|---|
| 100 | First ethylene-vinyl acetate layer | Ethylene-vinyl acetate: 100% | 25 |
| 110 | Polyethylene layer | Polyethylene: 100% | 25 |
| 120 | Second ethylene-vinyl acetate layer | Ethylene-vinyl acetate: 100% | 25 |

The percentage by weight of polyethylene in film 9 is approximately 33 wt.% and the percentage by weight of ethylene-vinyl acetate in film 9 is approximately 66 wt.%.

As for film 8 above, the trilayer film 9 is also produced by multilayer cast extrusion, which involves providing in polymer melt form: first and second ethylene-vinyl acetate layer polymer compositions corresponding to layers 100 and 120, and a polyethylene layer polymer composition corresponding to layer 110. The polymer compositions for the layers of the film are then co-extruded to form the trilayer film 9.

### Alternative trilayer laminate films

Tables 3 and 4 below describe examples of alternative trilayer laminate films which could also be used in other embodiments of urine collection bags and catheter sleeves of the invention.

Table 3 describes a trilayer film particularly suited for use in construction of a catheter sleeve of the invention, and Table 4 describes a film particularly suited for use in construction of a urine collection bag of the invention. The order of arrangement of the layers of the films are as described in the respective tables.

**Table 3**

| **Layer No.** | **Layer type** | **Layer materials** | **Layer thickness (µm)** |
|---|---|---|---|
| 200 | First ethylene-vinyl acetate layer | Ethylene-vinyl acetate: 100% | 15 |
| 210 | Polyethylene layer | Polyethylene: 100% | 20 |
| 220 | Second ethylene-vinyl acetate layer | Ethylene-vinyl acetate: 100% | 15 |

**Table 4**

| **Layer No.** | **Layer type** | **Layer materials** | **Layer thickness (µm)** |
|---|---|---|---|
| 300 | First ethylene-vinyl acetate layer | Ethylene-vinyl acetate: 100% | 22.5 |
| 310 | Polyethylene layer | Polyethylene: 100% | 30 |
| 320 | Second ethylene-vinyl acetate layer | Ethylene-vinyl acetate: 100% | 22.5 |

The percentage by weight of polyethylene in both films is approximately 40 wt.% and the percentage by weight of ethylene-vinyl acetate is approximately 60 wt.%.

Tables 5 and 6 below describe two further examples of alternative trilayer laminate films which could also be used in other embodiments of urine collection bags and catheter sleeves of the invention.

Table 5 describes a trilayer film particularly suited for use in construction of a catheter sleeve of the invention, and Table 6 describes a film particularly suited for use in construction of a urine collection bag of the invention. The order of arrangement of the layers of the films are as described in the respective tables.

**Table 5**

| **Layer No.** | **Layer type** | **Layer materials** | **Layer thickness (µm)** |
|---|---|---|---|
| 400 | First ethylene-vinyl acetate layer | Ethylene-vinyl acetate: 100% | 20 |
| 410 | Polyethylene layer | Polyethylene: 100% | 5 |
| 420 | Second ethylene-vinyl acetate layer | Ethylene-vinyl acetate: 100% | 20 |

**Table 6**

| **Layer No.** | **Layer type** | **Layer materials** | **Layer thickness (µm)** |
|---|---|---|---|
| 500 | First ethylene-vinyl acetate layer | Ethylene-vinyl acetate: 100% | 40 |
| 510 | Polyethylene layer | Polyethylene: 100% | 10 |
| 520 | Second ethylene-vinyl acetate layer | Ethylene-vinyl acetate: 100% | 40 |

The percentage by weight of polyethylene in both films is approximately 11 wt.% and the percentage by weight of ethylene-vinyl acetate is approximately 89 wt.%.

### Results

Trilayer laminate films of the invention described above provide several advantages over traditional materials. The combination of layers employed confers exceptional strength, even though the films are thin - e.g. for films 8 and 9, having respective film thicknesses of 60 microns and 75 microns. The fact that such thin layers of film can be used successfully significantly reduces the amount of plastic required in manufacture of the films, which helps minimise plastic waste and contributes to environmental sustainability.

The films are also lightweight despite their high strength, which reduces unnecessary load that a user must carry when performing catheterisation. In the case of film 9, for instance, the film 9 allows for construction of a lightweight urine collection bag 3 that can still hold large amounts of urine effectively.

The films are also relatively quiet when handled and do not produce significant rustling or other disruptive sounds during movement, unlike various conventional materials previously used to make catheter kit components. This significantly enhances patient comfort and privacy, especially in the case of self-catheterisation.

The films also provide excellent resistance to discolouration when subjected to sterilisation processes. Sterilisation is a critical step to maintain the hygiene and safety of catheter kit components and the films preserve their appearance and integrity even after repeated sterilisation cycles.

The above embodiments are described by way of example only. Many variations are possible without departing from the scope of the invention as defined in the appended claims.

## Claims

1. A urine collection bag (3) comprising a cavity (17) for containing urine, the bag (3) comprising a trilayer laminate film (9), **characterised in that** the film (9) comprises in order: a first ethylene-vinyl acetate layer (100), a polyethylene layer (110), and a second ethylene-vinyl acetate layer (120).

2. A urine collection bag (3) as claimed in claim 1, wherein the cavity (17) of the bag (3) is defined by the trilayer film (9).

3. A urine collection bag (3) as claimed in any preceding claim, wherein the bag (3) comprises a front wall (15) and a rear wall (16), wherein the front and rear walls (15,16) are sealed together to form the cavity (17) of the bag (3) therebetween, and wherein the front and rear wall (15,16) comprise the trilayer film (9).

4. A urine collection bag (3) as claimed in any preceding claim, wherein at least one layer of the film (9) has a thickness of between 10-40 microns, optionally wherein:
at least one ethylene-vinyl acetate layer (100,120) has a thickness of between 10-40 microns; and/or
the polyethylene layer (110) has a thickness of between 10-40 microns.

5. A urine collection bag (3) as claimed in any preceding claim, wherein the film (9) has a total thickness of between 50-100 microns.

6. A catheter sleeve (2) comprising a trilayer laminate film (8) **characterised in that** the film comprises in order: a first ethylene-vinyl acetate layer (10), a polyethylene layer (11), and a second ethylene-vinyl acetate layer (12).

7. A catheter sleeve (2) as claimed in claim 6, wherein at least one layer of the film (8) has a thickness of between 5-30 microns, optionally wherein:
at least one ethylene-vinyl acetate layer (10,12) has a thickness of between 5-30 microns; and/or
the polyethylene layer (11) has a thickness of between 5-30 microns.

8. A catheter sleeve (2) as claimed in claim 6 or claim 7, wherein the film (8) has a total thickness of between 40-70 microns.

9. A urine collection bag (3) as claimed in any one of claims 1 to 5 or a catheter sleeve (2) as claimed in any one of claims 6 to 8, wherein the polyethylene layer (11,110) of the film (8,9) comprises polyethylene in a total amount of at least 80 wt.% of the polyethylene layer (11,110), and preferably the polyethylene layer (11,110) is entirely made of polyethylene, and wherein the polyethylene layer (11,110) of the film (8,9) optionally comprises ethylene-vinyl acetate in a total amount of less than 10 wt. % of the polyethylene layer (11,110), and preferably the polyethylene layer (11,110) does not comprise ethylene-vinyl acetate.

10. A urine collection bag (3) as claimed in any one of claims 1 to 5 or claim 9 or a catheter sleeve (2) as claimed in any one of claims 6 to 9, wherein at least one ethylene-vinyl acetate layer (10,12,100,120) of the film (8,9) comprises ethylene-vinyl acetate in a total amount of at least 95 wt.% of the ethylene-vinyl acetate layer (10,12,100,120), and preferably the at least one ethylene-vinyl acetate layer (10,12,100,120) is entirely made of ethylene-vinyl acetate, and wherein at least one ethylene-vinyl acetate layer (10,12,100,120) of the film (8,9) optionally comprises polyethylene in a total amount of less than 10 wt.% of the ethylene-vinyl acetate layer (10,12,100,120), and preferably the at least one ethylene-vinyl acetate layer (10,12,100,120) does not comprise polyethylene.

11. A urine collection bag (3) as claimed in any one of claims 1 to 5 or in any one of claims 9 to 10 or a catheter sleeve (2) as claimed in any one of claims 6 to 10, wherein both the first and second ethylene-vinyl acetate layers (10,12,100,120) of the film (8,9) have the same thickness, and optionally all three layers of the trilayer film (8,9) have the same thickness.

12. A urine collection bag (3) as claimed in any one of claims 1 to 5 or in any one of claims 9 to 11 or a catheter sleeve (2) as claimed in any one of claims 6 to 11, wherein the film (8,9) comprises:
polyethylene in a total amount of between 5-50 wt.%, or preferably between 10-45 wt.%; and/or
ethylene-vinyl acetate in a total amount of between 50-95 wt.%, or preferably between 55-90 wt. %.

13. A urine collection bag (3) as claimed in any one of claims 1 to 5 or in any one of claims 9 to 12 or a catheter sleeve (2) as claimed in any one of claims 6 to 12, wherein the polyethylene layer (11,110) comprises low-density polyethylene.

14. A urinary catheter kit (1) comprising: a catheter (4) comprising a proximal end (5) for insertion into the body and a distal end (6); a urine collection bag (3) according to any one of claims 1 to 5 or 9 to 13, further comprising an inlet (7) adapted for fluid communication with the catheter distal end (6); and a catheter sleeve (2) according to any one of claims 6 to 13, configured to enclose at least part of the catheter length from the proximal end (5) to the distal end (6).

15. A method of manufacturing a urine collection bag (3) comprising the steps of:
a. Providing a front wall (15) and a rear wall (16), wherein the front and rear walls (15,16) comprise a trilayer barrier film (9) **;** and
b. Sealing the front and rear walls (15,16) together to form a cavity (17) therebetween for containing urine;
wherein the method further comprises providing an inlet (7) for receiving urine in the front or rear wall (15,16) before or after step (b)
**characterised in that** film (9) comprising in order: a first ethylene-vinyl acetate layer (100), a polyethylene layer (110), and a second ethylene-vinyl acetate layer (120).

## Patentansprüche

1. Urinsammelbeutel (3), aufweisend einen Hohlraum (17) zum Enthalten von Urin, wobei der Beutel (3) eine dreischichtige Laminatfolie (9) aufweist, **dadurch gekennzeichnet, dass** die Folie (9) in dieser Reihenfolge aufweist: eine erste Ethylen-Vinylacetat-Schicht (100), eine Polyethylen-Schicht (110) und eine zweite Ethylen-Vinylacetat-Schicht (120).

2. Urinsammelbeutel (3) nach Anspruch 1, wobei der Hohlraum (17) des Beutels (3) durch die dreischichtige Folie (9) definiert ist.

3. Urinsammelbeutel (3) nach einem der vorhergehenden Ansprüche, wobei der Beutel (3) eine Vorderwand (15) und eine Rückwand (16) aufweist, wobei die Vorder- und Rückwände (15,16) miteinander versiegelt sind, um den Hohlraum (17) des Beutels (3) dazwischen zu bilden, und wobei die Vorder- und Rückwände (15,16) die dreischichtige Folie (9) aufweisen.

4. Urinsammelbeutel (3) nach einem der vorhergehenden Ansprüche, wobei mindestens eine Schicht der Folie (9) eine Dicke zwischen 10-40 Mikrometern aufweist, optional wobei:
mindestens eine Ethylen-Vinylacetat-Schicht (100,120) eine Dicke zwischen 10-40 Mikrometern aufweist; und/oder
die Polyethylen-Schicht (110) eine Dicke zwischen 10-40 Mikrometern aufweist.

5. Urinsammelbeutel (3) nach einem der vorhergehenden Ansprüche, wobei die Folie (9) eine Gesamtdicke zwischen 50-100 Mikrometern aufweist.

6. Katheterhülse (2), aufweisend eine dreischichtige Laminatfolie (8), **dadurch gekennzeichnet, dass** die Folie in dieser Reihenfolge aufweist: eine erste Ethylen-Vinylacetat-Schicht (10), eine Polyethylen-Schicht (11) und eine zweite Ethylen-Vinylacetat-Schicht (12).

7. Katheterhülse (2) nach Anspruch 6, wobei mindestens eine Schicht der Folie (8) eine Dicke zwischen 5-30 Mikrometern aufweist, optional wobei:
mindestens eine Ethylen-Vinylacetat-Schicht (10,12) eine Dicke zwischen 5-30 Mikrometern aufweist; und/oder
die Polyethylen-Schicht (11) eine Dicke zwischen 5-30 Mikrometern aufweist.

8. Katheterhülse (2) nach Anspruch 6 oder Anspruch 7, wobei die Folie (8) eine Gesamtdicke zwischen 40-70 Mikrometern aufweist.

9. Urinsammelbeutel (3) nach einem der Ansprüche 1 bis 5 oder eine Katheterhülse (2) nach einem der Ansprüche 6 bis 8, wobei die Polyethylen-Schicht (11,110) der Folie (8,9) Polyethylen in einer Gesamtmenge von mindestens 80 Gew.-% der Polyethylen-Schicht (11,110) aufweist, und vorzugsweise die Polyethylen-Schicht (11, 110) vollständig aus Polyethylen hergestellt ist, und wobei die Polyethylen-Schicht (11,110) der Folie (8,9) optional Ethylen-Vinylacetat in einer Gesamtmenge von weniger als 10 Gew.-% der Polyethylen-Schicht (11, 110) aufweist, und vorzugsweise die Polyethylen-Schicht (11, 110) kein Ethylen-Vinylacetat aufweist.

10. Urinsammelbeutel (3) nach einem der Ansprüche 1 bis 5 oder Anspruch 9 oder eine Katheterhülse (2) nach einem der Ansprüche 6 bis 9, wobei mindestens eine Ethylen-Vinylacetat-Schicht (10,12,100,120) der Folie (8,9) Ethylen-Vinylacetat in einer Gesamtmenge von mindestens 95 Gew.-% der Ethylen-Vinylacetat-Schicht (10,12,100,120) aufweist, und vorzugsweise die mindestens eine Ethylen-Vinylacetat-Schicht (10,12,100,120) vollständig aus Ethylen-Vinylacetat hergestellt ist, und wobei mindestens eine Ethylen-Vinylacetat-Schicht (10,12,100,120) der Folie (8,9) optional Polyethylen in einer Gesamtmenge von weniger als 10 Gew.-% der Ethylen-Vinylacetat-Schicht (10,12,100,120) aufweist, und vorzugsweise die mindestens eine Ethylen-Vinylacetat-Schicht (10,12,100,120) kein Polyethylen aufweist.

11. Urinsammelbeutel (3) nach einem der Ansprüche 1 bis 5 oder nach einem der Ansprüche 9 bis 10 oder eine Katheterhülse (2) nach einem der Ansprüche 6 bis 10, wobei sowohl die erste als auch die zweite Ethylen-Vinylacetat-Schichten (10,12,100,120) der Folie (8,9) dieselbe Dicke aufweisen, und optional alle drei Schichten der dreischichtigen Folie (8,9) dieselbe Dicke aufweisen.

12. Urinsammelbeutel (3) nach einem der Ansprüche 1 bis 5 oder nach einem der Ansprüche 9 bis 11 oder eine Katheterhülse (2) nach einem der Ansprüche 6 bis 11, wobei die Folie (8,9) aufweist:
Polyethylen in einer Gesamtmenge zwischen 5-50 Gew.-%, oder vorzugsweise zwischen 10-45 Gew.-%; und/oder
Ethylen-Vinylacetat in einer Gesamtmenge zwischen 50-95 Gew.-%, oder vorzugsweise zwischen 55-90 Gew.-%.

13. Urinsammelbeutel (3) nach einem der Ansprüche 1 bis 5 oder nach einem der Ansprüche 9 bis 12 oder eine Katheterhülse (2) nach einem der Ansprüche 6 bis 12, wobei die Polyethylen-Schicht (11,110) Polyethylen niedriger Dichte aufweist.

14. Harnkatheter-Kit (1), aufweisend: einen Katheter (4), aufweisend ein proximales Ende (5) zum Einführen in den Körper und ein distales Ende (6); einen Urinsammelbeutel (3) nach einem der Ansprüche 1 bis 5 oder 9 bis 13, ferner aufweisend einen Einlass (7), der zur Fluidkommunikation mit dem distalen Katheterende (6) angepasst ist; und eine Katheterhülse (2) nach einem der Ansprüche 6 bis 13, eingerichtet zum Umschließen mindestens eines Teils der Katheterlänge von dem proximalen Ende (5) bis zu dem distalen Ende (6).

15. Verfahren zum Herstellen eines Urinsammelbeutels (3), aufweisend die Schritte:
a. Bereitstellen einer Vorderwand (15) und einer Rückwand (16), wobei die Vorder- und Rückwände (15,16) eine dreischichtige Barrierefolie (9) aufweisen;
b. Versiegeln der Vorder- und Rückwände (15,16) miteinander, um einen Hohlraum (17) dazwischen zum Enthalten von Urin zu bilden;
wobei das Verfahren ferner das Bereitstellen eines Einlasses (7) zum Empfangen von Urin in der Vorder- oder Rückwand (15,16) vor oder nach Schritt (b) aufweist, **dadurch gekennzeichnet, dass** Folie (9) in dieser Reihenfolge aufweist: eine erste Ethylen-Vinylacetat-Schicht (100), eine Polyethylen-Schicht (110) und eine zweite Ethylen-Vinylacetat-Schicht (120).

## Revendications

1. Un sac (3) de collecte d'urine comprenant une cavité (17) pour contenir de l'urine, le sac (3) comprenant un film (9) stratifié à trois couches, **caractérisé en ce que** le film (9) comprend dans l'ordre : une première couche (100) en éthylène-acétate de vinyle, une couche (110) en polyéthylène et une deuxième couche (120) en éthylène-acétate de vinyle.

2. Un sac (3) de collecte d'urine tel que revendiqué dans la revendication 1, dans lequel la cavité (17) du sac (3) est définie par le film (9) à trois couches.

3. Un sac (3) de collecte d'urine tel que revendiqué dans l'une quelconque des revendications précédentes, dans lequel le sac (3) comprend une paroi (15) avant et une paroi (16) arrière, dans lequel les parois (15, 16) avant et arrière sont scellées ensemble pour former la cavité (17) du sac (3) entre elles, et dans lequel les parois (15, 16) avant et arrière comprennent le film (9) à trois couches.

4. Un sac (3) de collecte d'urine tel que revendiqué dans l'une quelconque des revendications précédentes, dans lequel au moins une couche du film (9) a une épaisseur comprise entre 10 et 40 microns, dans lequel éventuellement :
au moins une couche (100, 120) en éthylène-acétate de vinyle a une épaisseur comprise entre 10 et 40 microns ; et/ou
la couche (110) de polyéthylène a une épaisseur comprise entre 10 et 40 microns.

5. Un sac (3) de collecte d'urine tel que revendiqué dans l'une quelconque des revendications précédentes, dans lequel le film (9) a une épaisseur totale comprise entre 50 et 100 microns.

6. Une gaine (2) de cathéter comprenant un film (8) stratifié à trois couches, **caractérisée en ce que** le film comprend dans l'ordre : une première couche (10) en éthylène-acétate de vinyle, une couche (11) en polyéthylène et une deuxième couche (12) en éthylène-acétate de vinyle.

7. Une gaine (2) de cathéter telle que revendiquée dans la revendication 6, dans laquelle au moins une couche du film (8) a une épaisseur comprise entre 5 et 30 microns, dans laquelle éventuellement :
au moins une couche (10, 12) en éthylène-acétate de vinyle a une épaisseur comprise entre 5 et 30 microns ; et/ou
la couche (11) en polyéthylène a une épaisseur comprise entre 5 et 30 microns.

8. Une gaine (2) de cathéter telle que revendiquée dans la revendication 6 ou la revendication 7, dans laquelle le film (8) a une épaisseur totale comprise entre 40 et 70 microns.

9. Un sac (3) de collecte d'urine tel que revendiqué dans l'une quelconque des revendications 1 à 5 ou une gaine (2) de cathéter telle que revendiquée dans l'une quelconque des revendications 6 à 8, dans lequel la couche (11, 110) en polyéthylène du film (8, 9) comprend du polyéthylène en une quantité totale d'au moins 80 % en poids de la couche (11, 110) en polyéthylène, et de préférence la couche (11, 110) en polyéthylène est faite entièrement en polyéthylène, et dans lequel la couche (11, 110) du film (8, 9) comprend éventuellement de l'éthylène-acétate de vinyle en une quantité totale de moins de 10 % en poids de la couche (11, 110) en polyéthylène, et de préférence la couche (11, 110) en polyéthylène ne comprend pas d'éthylène-acétate de vinyle.

10. Un sac (3) de collecte d'urine tel que revendiqué dans l'une quelconque des revendications 1 à 5 ou la revendication 9 ou une gaine (2) de cathéter telle que revendiquée dans l'une quelconque des revendications 6 à 9, dans lequel au moins une couche (10, 12, 100, 120) en éthylène-acétate de vinyle du film (8, 9) comprend de l'éthylène-acétate de vinyle en une quantité totale d'au moins 95 % en poids de la couche (10, 12, 100, 120) en éthylène-acétate de vinyle, et de préférence la au moins une couche (10, 12, 100, 120) en éthylène-acétate de vinyle est faite entièrement en éthylène-acétate de vinyle, et dans lequel au moins une couche (10, 12, 100, 120) en éthylène-acétate de vinyle du film (8, 9) comprend éventuellement du polyéthylène en une quantité totale de moins de 10 % en poids de la couche (10, 12, 100, 120) en éthylène-acétate de vinyle, et de préférence la au moins une couche (10, 12, 100, 120) en éthylène-acétate de vinyle ne comprend pas de polyéthylène.

11. Un sac (3) de collecte d'urine tel que revendiqué dans l'une quelconque des revendications 1 à 5 ou dans l'une quelconque des revendications 9 à 10 ou une gaine (2) de cathéter telle que revendiquée dans l'une quelconque des revendications 6 à 10, dans lequel, à la fois la première et la deuxième couches (10, 12, 100, 120) en éthylène-acétate de vinyle du film (8, 9) ont la même épaisseur, et éventuellement toutes les trois couches du film (8, 9) à trois couches ont la même épaisseur.

12. Un sac (3) de collecte d'urine tel que revendiqué dans l'une quelconque des revendications 1 à 5 ou dans l'une quelconque des revendications 9 à 11 ou une gaine (2) de cathéter telle que revendiquée dans l'une quelconque des revendications 6 à 11, dans lequel le film (8, 9) comprend :
du polyéthylène en une quantité totale comprise entre 5 et 50 % en poids, ou de préférence entre 10 et 45 % en poids ; et/ou
de l'éthylène-acétate de vinyle en quantité totale comprise entre 50 et 95 % en poids, ou de préférence entre 55 et 90 % en poids.

13. Un sac (3) de collecte d'urine tel que revendiqué dans l'une quelconque des revendications 1 à 5 ou dans l'une quelconque des revendications 9 à 12 ou une gaine (2) de cathéter telle que revendiquée dans l'une quelconque des revendications 6 à 12, dans lequel la couche (11, 110) de polyéthylène comprend du polyéthylène basse densité.

14. Une trousse (1) de cathéter urinaire comprenant : un cathéter (4) comprenant une extrémité (5) proximale pour insertion dans le corps et une extrémité (6) distale ; un sac (3) de collecte d'urine suivant l'une quelconque des revendications 1 à 5 ou 9 à 13, comprenant en outre une entrée (7) propre à une communication fluidique avec l'extrémité (6) distale du cathéter ; et une gaine (2) de cathéter suivant l'une quelconque des revendications 6 à 13, configurée pour enfermer au moins une partie de la longueur du cathéter de l'extrémité (5) proximale à l'extrémité (6) distale.

15. Un procédé de fabrication d'un sac (3) de collecte d'urine comprenant les stades de :
a. se procurer une paroi (15) avant et une paroi (16) arrière, dans lequel les parois (15, 16) avant et arrière comprennent un film (9) barrière à trois couches ;
b. sceller les parois (15, 16) avant et arrière ensemble pour former entre elles une cavité (17) pour contenir de l'urine ;
dans lequel le procédé comprend en outre ménager une entrée (7) de réception d'urine dans la paroi (15, 16) avant ou arrière avant ou après le stade b),
**caractérisé en ce que** le film (9) comprend dans l'ordre : une première couche (100) en éthylène-acétate de vinyle, une couche (110) en polyéthylène et une deuxième couche (120) en éthylène-acétate de vinyle.
